# EUROPEAN PATENT SPECIFICATION

(11) **EP 0 714 313 B1**
(45) Date of publication and mention of the grant of the patent: **15.01.2003**
(21) Application number: 94922400.0
(22) Date of filing: 25.07.1994
(51) Int. Cl.: A61M 15/00

(54) **POWDER INHALER**
PULVER-INHALATOR
INHALATEUR A POUDRE

(30) Priority: 30.07.1993 SE 9302550
(43) Date of publication of application: 05.06.1996
(60) Divisional application: 02023463.9
(73) Proprietor: Aventis Pharma Limited, West Malling, Kent ME19 4AH (GB)
(72) Inventor: Hörlin, Ernst, 436 39 Askim (SE)
(74) Representative: Harrison, Michael Charles
(86) International application number: PCT/SE94/00704
(87) International publication number: WO 95/003846

(56) References cited:
- EP-A- 0 407 028
- EP-A- 0 504 459
- WO-A-90/15635
- FR-A- 2 352 556
- SE-B- 428 426
- US-A- 4 841 964

## Description

### Field of the Invention

The present invention relates to an inhaler in accordance with the first part of claim 1. In particular the invention relates to an inhaler for use with the inhalation of powdered medicinal substances.

### Background to the Invention

Various types of inhaler are known and widely used on the market. For example, many asthma sufferers regularly use a spray inhaler comprising a gas propellant which is stored in a metal container containing additionally a medicinal substance to be inhaled to ease the cause and or symptoms of the affliction. The metal container is connected in use to a hollow plastic carrier which at one end has a mouthpiece for the user. The device is operated by pressing the metal container inwardly with respect to said plastic carrier to thus release a pressurised dosage of medicine.

Such devices are however bad for the environment due to the use of propellants and other carrier gases. Additionally the amount of medicament which is inhaled comes from a store of medicine in the container suitable for many inhalations, such that considerable problems arise concerning the amount of each dose inhaled due to incomplete or uneven mixing of the medicament, or due to variations in the available gas pressure. Further dosage anomalies may also occur if the inhaler has been allowed to stand for a long period of time between uses. Moreover such inhalers are relatively bulky and expensive.

Also known are inhalers which have a body portion containing an impeller blade or fan incorporated therein and further including a compartment for the introduction of a medicament. On operation, said medicament is ejected by the action of the rising air flow caused by the fan.

Such inhalers have the disadvantage that an uneven flow often results and the construction of the device makes it relatively expensive, bulky and prone to breakage or malfunction.

A further known inhaler is disclosed in US-A-4 069 819. The inhaler device of this document however presents difficulties in normal operation because powder is withdrawn slowly from the capsule within the capsule chamber, partly due to the small diameter of the holes which are possible in the capsule due to the design of the device and partly due to the limited movement of the capsule in the chamber. The capsule is restrained in the longitudinal direction by an intermediate wall containing a plurality of holes and comprising a semi-spherical lower surface which is on a wider arc than that of the capsule upper end to thereby allow rotational procession of the capsule thereagainst without substantial hindrance, whilst still allowing powder to reach the user.

The powder entering the mouthpiece is thus confined to a substantially laminar flow such that the dispersion/separation thereof is minimal and the sucking effort required will often be quite large. Moreover one or more of the plurality of passages will often be blocked by the capsule leading to particle build-up and clogging.

US-A-4 841 964 discloses a device having the features defined in the first part of claim 1. This document describes an inhaler including a chamber having a ball movable therein. The ball is sized to have a minimal amount of play with respect to the track and is arranged to undergo orbital motion in the track under the effect of air which enters via a single tangential air intake. A medicinal substance is applied either to the ball or to the track. Upon sucking, a substantially laminar air flow results which gives rise to the required orbital motion of the ball around the track. Fragmentation of the applied medicinal substance as a result of the shear and compressive forces between the track and the ball thereby takes place. The medicinal substance which is released by fragmentation passes with the air from the upper part of the track through a discharge system comprising a restricted area formed between a central projection and a cover sheet. The air and medicinal substance then subsequently pass through an air outlet opening arranged in the cover sheet. A mouthpiece may be fitted on the cover sheet so as to communicate with the outlet opening.

### Object of the Invention

The object of the present invention is therefore to overcome the problems of the prior art devices by providing an inhaler which is simple and reliable for use with powder substances and which further provides good dispersion characteristics so that the powder more easily reaches the lungs.

A still further object of the invention is to provide a device which is relatively inexpensive and thus readily disposable.

Further objects and advantages of the invention will become apparent to the skilled man upon studying the following description and drawings of a preferred embodiment.

### Summary of the Invention

The invention has the features defined in claim 1 appended hereto. Preferred features of the invention are defined in the dependent claims.

By the use of the feature of claim 1 that a single restriction is present in combination with more than one entrance passageway, the velocity upon exiting the chamber will increase and a swirling effect upon air passing from the chamber into the tubular member can be maintained which leads to better dispersion characteristics.

### Brief description of the drawings

- Fig. 1: shows a cross-section through a device having a single entrance passageway; This device is for explication and does not fall under claim 1.
- Fig. 2: shows a cross-section through the device taken along line II-II of Fig. 1;
- Fig. 3: shows a cross-section through a first embodiment of the invention;
- Fig.4: shows a cross-section taken along line IV-IV in Fig.3
- Fig.5: shows a cross-section taken along line V-V in Fig.3
- Fig. 6: shows a second embodiment of the invention;
- Fig.7: shows a cross-sectional view of the device according to Fig. 3 in the position of use within the mouth of a user, and
- Fig.8: shows an exploded view of a device according to the invention fitted with a capsule magazine.

### Description of preferred embodiments

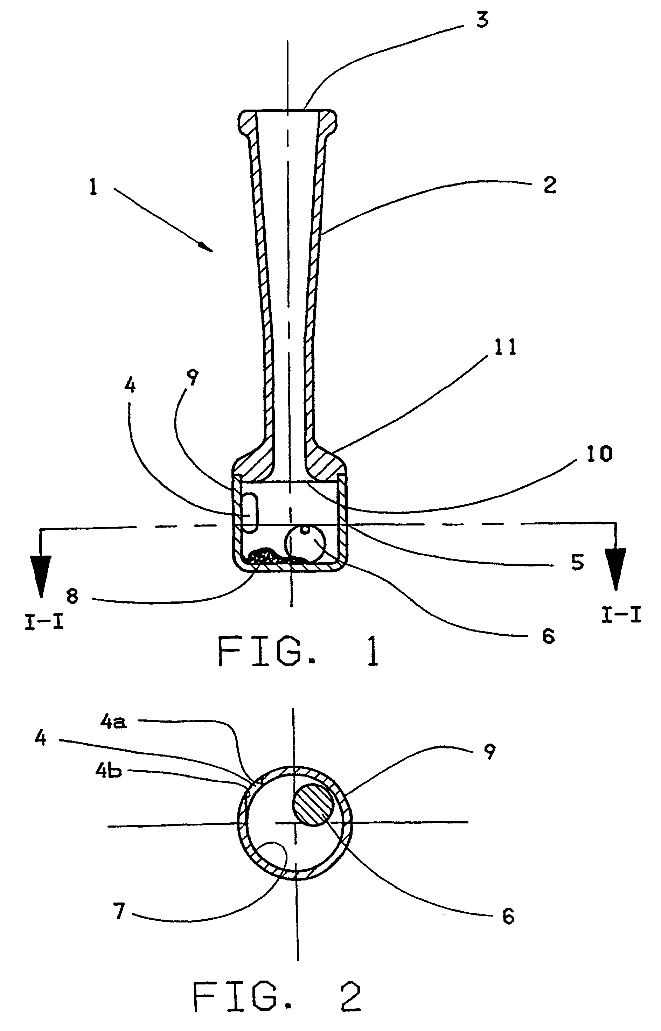

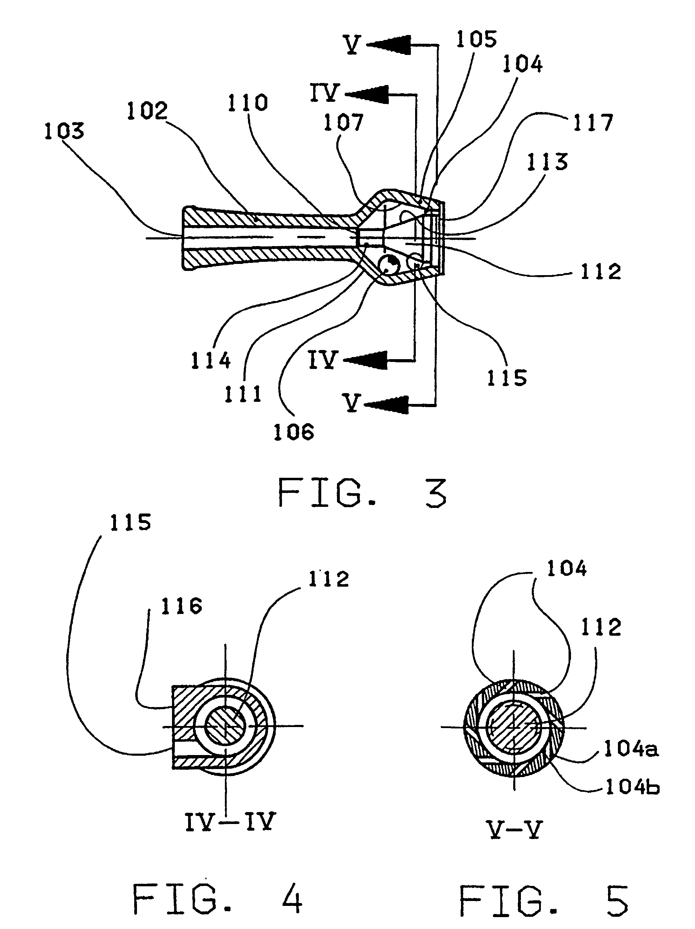

Fig.1 shows a two-part inhaler device 1 which comprises a tubular member 2 which has a first opening 3 at one end and a second opening 10 at the other end connected to a chamber 5. In between the first opening 3 and the planar end wall of the chamber 5 the device 2 is hollow as shown in the figures.

An opening 4 is also provided in the chamber 5 and serves as a passageway to connect the inside-of the chamber 5 with the surrounding air.

The tubular member 2 of the device 1 is formed as a mouthpiece designed to be held within the lips of a user, the mouthpiece preferably being inserted into the user's mouth so that the lips of the user will rest on the smooth curved surface 11.

A freely movable element 6, preferably in the form of a sphere, is positioned inside the chamber.

Whilst a two-piece device is shown, a one-piece device or a device having more pieces is also possible. Similarly the material may be chosen as required by the circumstances. For most applications it will be possible to construct the device from plastics material such as e.g. transparent plastics material or PVC. The number of passageways are increased, in order to provide a device in accordance with the invention.

The passageway 4, as best seen in Fig. 2 consists of an opening formed in the wall of the chamber 5. The opening 4 is formed appropriately so as to cause the air entering the chamber to swirl around the chamber. In the shown embodiment the sides 4a, 4b are offset from the central rotational axis of the chamber such that the outer side 4b is substantially tangential to the inner wall 7 of the chamber. The inner wall is substantially cylindrical and circular, although it may be foreseen with projections or another type of surface profile (e.g. a roughened surface).

The chamber 5, in use, will contain a quantity of medicament substance 8 in dry powder form, which is to be inhaled by the user who will put the mouthpiece to his lips and suck (see also Fig. 7).

Due to the sucking action, air will be drawn in through the opening 4 in the chamber (similar to an inverse whistle) and, due to the orientation of the opening 4, will circulate around the chamber 4 thus causing a swirling of the air therein. The element 6 will be caused to spin and to move under this swirling action of the air and will thus vibrate inside the chamber, in turn causing the powdered medicament to be taken along with the swirling air towards the opening 3 from where it exits into the user.

Only a short suck, requiring small sucking effort, is required on the mouthpiece 3 to give the element 6 a high velocity around the chamber and thus to effect dispersal of the powder as required for inhalation. Due to the presence of only one restriction 10, in this case at the interface between the member 2 and the chamber 5, the turbulence and swirling of the air and powder will be maintained when passing from the chamber 5 to the tubular member 2. The restriction 10 will also cause the velocity of the air to increase as it exits the chamber which will increase inter-particle bombardment leading to a finer particle size of the powder substance 8. In this way any lumps of powder will be more uniformly dispersed.

If desired, the effect of the vibrations of the element 6 may be enhanced by providing roughening or other projecting members (e.g. ribs or serrations) on the inner surface 7.

In the invention, the element 6 may itself contain the medicinal substance for inhalation, appropriate outlets being provided in the element.

With the device according to the invention, an efficient and environmentally-friendly inhaler is produced which is thus both simple and reliable.

Due to the relative cheapness of production of such a device, the device has suitability as a disposable device for once-only use, containing the quantity of medicament required for said one use. Typically such a quantity might be between 5 and 30mg, typically 20mg, although smaller or larger doses are possible. If the device is made in two parts as shown for example, the part 2 could be re-usable and part 9, corresponding to the chamber section 5, could be a disposable part. For reasons of hygiene, suitable removable or pierceable means to cover the opening 4 and the opening from the chamber into the part 2 at the restriction should be provided.

A first embodiment of the invention is shown in Fig.3, like features being denoted with the same reference numerals as in the previous figure but increased by 100.

The inhaler device of this embodiment is made in one piece, the member 102 and the chamber 105 being combined and having a different shape. The shape of the chamber portion 105 is such that, starting from the end face 117 of the chamber and moving in a direction towards the member 102, the inner wall 107 of the chamber 105 has a section "a" defining an increasing cross-sectional area followed by a section "b" of decreasing cross-sectional area. This arrangement has the particular effect that air drawn in through the passageways 104 first undergoes a decrease in velocity due to the increasing cross-section in section "a" followed by a transition at the interface between sections "a" and "b" to an increasing velocity. By appropriate choice of angle of inclination of the sections "a" and "b", the air may also be caused to separate from the sidewall 107 to form eddy currents. The net effect of this is to cause a large amount of turbulence in the air in the chamber which will increase inter-particle bombardment and thus increase the uniformity of dispersion. The effect of this turbulence is enhanced by the use of a further element 106, such as a ball (as explained with reference to Fig.1).

The device is further fitted with a central core element 112 having preferably a trunconic portion 113 joined to a circular cylindrical portion 114 of constant diameter. The core element 112 may be formed as one piece with the end wall 117 or attached thereto as appropriate. The portion 114 extends up to the area of the restriction 110.

When air is sucked in through passageways 104, the turbulent effect caused by the sidewall shape is enhanced by the shape of the core element 112 which allows only a small area of the chamber to be "active" for air movement whilst still providing space for movement of the element 106 therein.

As can be seen from Fig.3, the portion 114 extends up to the restriction 110 in such a way that a single, thin annular restriction is present around the outside of the core element 112. This further enhances the velocity increase at this point which gives a strong rotation of the air and powder substance at this location, in turn leading to further inter-particle bombardment (thus better uniformity of dispersion and lower particle size) and rotation along the member 2. Clogging at the restriction 110 is not a problem due to the high air velocity and at this point.

Thus in normal use, powder particles which are drawn into the swirling air will undergo the above described velocity changes of the air current due to their relatively low mass and will finely and uniformly disperse into the swirling air. Upon passing the restriction 110 the particles will then separate further along the gradually widening tubular element 2 and their velocity will decrease, which is important in order to allow the powder substance to be transported all the way to the user's lungs.

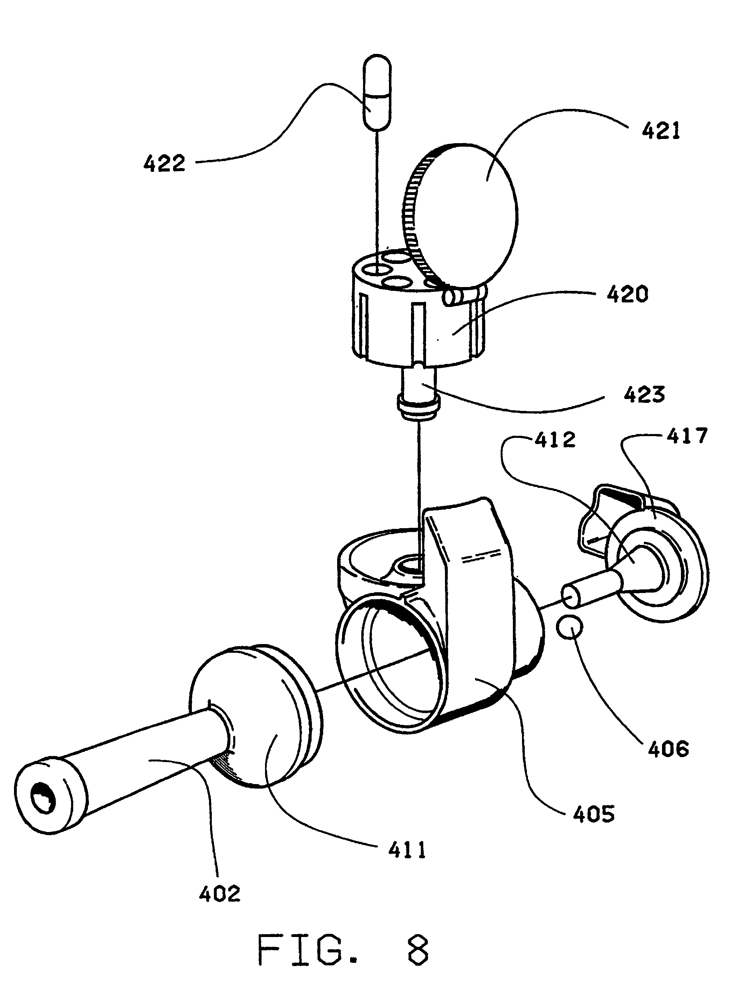

As also shown in Fig.4, the inhaler device is provided with a further opening 115 which serves as an inlet for the powder substance to be inhaled. The powder substance would normally enter through the opening 115 from an external magazine (see Fig.8), the powder being able to enter the chamber either before or during inhalation by the user.

As can be seen in Fig.4, the powder is also arranged to enter tangentially into the chamber, the magazine device or the like being positioned against the flat abutment face 116 of the chamber wall.

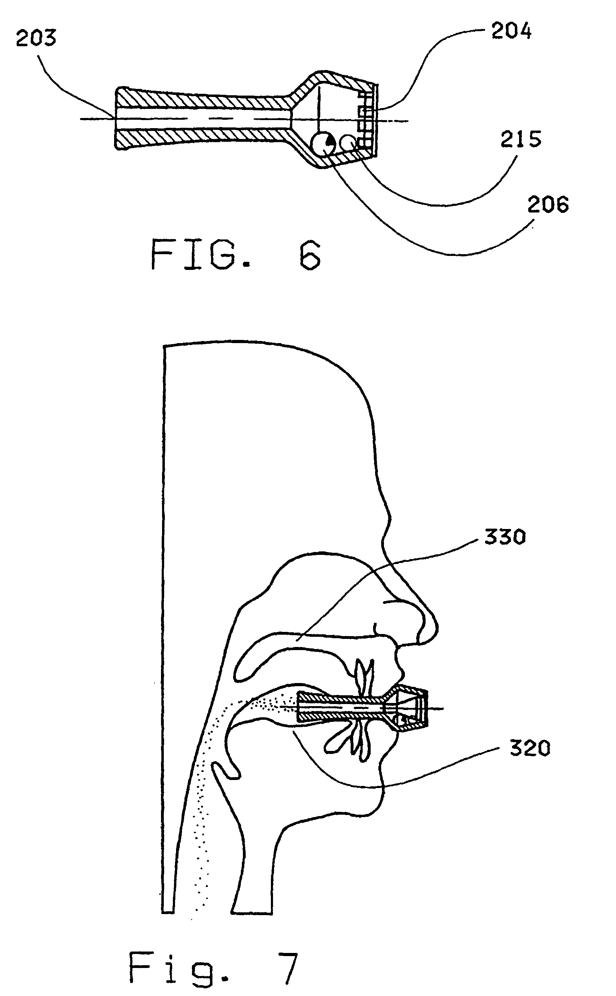

Fig. 5 shows a cross-section of the lower end of the chamber 105 with eight passageways 104 arranged to allow air entry.

Fig.6 depicts the same type of device as described with reference to Fig.3, although with no central core element 112 but having a ball element 206 arranged for free rotation when air is sucked in through passageway 204 due to suction effort at 203. A magazine may be attached to allow powder entry through hole 215.

Fig.7 depicts the preferred mode of use of the device, the lips of the user being placed against the lip abutment surface 111 and the opening 203 being positioned far enough back inside the mouth so that negligible powder is lost in the oral cavity (e.g. to the tongue 320 or palate 330) before entering the lungs. This has the further advantage that the taste sensors of the tongue are to a great extent unexposed to the medicinal substance which is relatively slow moving at this point.

In Fig.8 an embodiment fitted with a capsule magazine is depicted as an exploded view. The elements correspond basically to the elements shown in Fig. 3, although the numerals are in a 400 series instead of 100 series. Thus no detailed explanation of operation is required since this will be the same as for previous embodiments.

The tubular member 402 and lip abutment surface 411 are formed as a one piece moulding which is attached to chamber 405 by a push fit or the like. A ball 406 is contained within the chamber 405 and the chamber is closed at its lower end by an end face 417 having integral core element 412. A magazine containing capsules 422, which in turn contain a quantity of powder substance (preferably equal to one dose), is attached by means of pipe 423 to the housing of chamber 405 and through which powder from a ruptured capsule can be transported. The actual details of the magazine are unimportant to the invention, any suitable type of magazine being possible. In the shown embodiment however the magazine is provided with a flip lid 421 attached by a hinge to the magazine body 420.

The relative sizes, weights and materials of the device are largely a matter of choice according to the circumstances and can be varied within large limits. One example of dimensions for the device may typically be where the length of the mouthpiece or tubular member from the opening 3 to the lip abutment surface 11 is between about 2 to 6cm, with a chamber 5 diameter of between about 10mm and 20mm, preferably about 16 to 18mm. With such dimensions, the width of the opening 4 between side faces 4a and 4b would typically be about 2 to 3mm and the element 6 could be a sphere of about 3mm radius. Clearly such dimensions provide a slim and compact unit.

When the device is made of clear plastics, the additional advantage obtained would be that the user can see whether all the medicament has been inhaled or not.

Whilst preferred embodiments of the invention have been described above it is clear that many variations of the invention are possible within the scope of the claims appended hereto.

## Claims

1. An inhaler device for powder substances, comprising a tubular member (102, 402) connected to a chamber (105, 405) containing a medicament powder (8), said tubular member being provided with a first opening (103) at one end, and wherein said chamber (105, 405) further contains an element (106, 206, 406), preferably a sphere, which is sized to occupy a minor portion of said chamber (105, 405), whereby said element (106, 206, 406) is sized to be freely movable within said chamber, **characterized in that** a plurality of entrance passageways (104, 204) is provided in said chamber (105, 405), said plurality of entrance passageways (104, 204) being arranged substantially tangentially with respect to said chamber (105, 405) so as to cause air sucked therethrough to swirl around said chamber, and wherein a single restriction (110) is arranged in the direction of inhalation between the first opening (103, 203) and said plurality of entrance passageways (104, 204).

2. An inhaler device according to claim 1, **characterized in that** said chamber (105, 405) has a sidewall which is shaped so as to provide the chamber with a first portion (a) having increasing cross-sectional area in a direction towards said restriction and a second portion (b) with decreasing cross-sectional area in a direction towards said restriction (110).

3. An inhaler device according to any one of claims 1 or 2, **characterized in that** said chamber has an inner wall (107) which is substantially circular in cross-section.

4. An inhaler device according to any preceding claim, **characterized in that** a core element (112, 412) is arranged within said chamber, said core element extending between an end wall (117, 417) of said chamber to a position proximate the single restriction (110).

5. An inhaler device according to claim 4, **characterized in that** said core element has a trunconic portion (113) with its large end close to the end wall (117, 417) of said chamber and a constant-section cylindrical portion (114) extending from the smaller end of said trunconic portion up to the area of the restriction (110).

6. An inhaler device according to any preceding claim, **characterized in that** a magazine (420, 421) for carrying a plurality of powder doses, each preferably contained within a rupturable capsule (422), is attached to said chamber, and **in that** said magazine is connected to the interior of said chamber by means of an inlet opening provided in the wall of said chamber (105, 405).

7. An inhaler device according to any preceding claim, **characterized in that** at least said chamber (105, 405) of said inhaler device is made of transparent material.

8. An inhaler device according to any preceding claim, **characterized in that** a protruding portion (111, 411) is provided on the exterior of the inhaler device at a distance of between about 2 cm and 6 cm from said first opening (103, 203).

9. An inhaler device according to any one of claims 1 to 8, **characterized in that** said freely movable element (106, 206, 406) contains a substance in powder form.

## Patentansprüche

1. Inhaliervorrichtung für pulverförmige Substanzen, mit einem rohrförmigen Element (102, 402), das mit einer Kammer (105, 405) verbunden ist, die ein Heilmittelpulver (8) beinhaltet, wobei das rohrförmige Element mit einer ersten Öffnung (103) an einem Ende versehen ist und wobei die Kammer (105, 405) des weiteren ein Element (106, 206, 406), vorzugsweise eine Kugel, beinhaltet, die derart bemessen ist, daß sie einen kleineren Bereich der Kammer (105, 405) einnimmt, wobei das Element (106, 206, 406) so bemessen ist, daß es innerhalb der Kammer frei beweglich ist, **dadurch gekennzeichnet, daß** mehrere Eintrittsdurchgänge (104, 204) in der Kammer (105, 405) vorgesehen sind, wobei die mehreren Eintrittsdurchgänge (104, 204) im wesentlichen tangential in Bezug auf die Kammer (105, 405) angeordnet sind, und dadurch bewirken, daß durch sie gesaugte Luft um die Kammer wirbelt und wobei eine einzelne Durchlaßbegrenzung (110) in Richtung der Inhalation zwischen der ersten Öffnung (103, 203) und den mehreren Eintrittsdurchgängen (104, 204) angeordnet ist.

2. Inhaliervorrichtung nach Anspruch 1, **dadurch gekennzeichnet, daß** die Kammer (105, 405) eine Seitenwand hat, welche derart gestaltet ist, daß sie die Kammer mit einem ersten Abschnitt (a) mit einem zunehmenden Querschnittsbereich in Richtung der Durchlaßbegrenzung und einem zweiten Abschnitt (b) mit abnehmendem Querschnittsbereich in Richtung der Durchlaßbegrenzung (110), versieht.

3. Inhaliervorrichtung nach einem der Ansprüche 1 oder 2, **dadurch gekennzeichnet, daß** die Kammer eine Innenwand (107) hat, deren Querschnitt im wesentlichen kreisförmig ist.

4. Inhaliervorrichtung nach einem der vorstehenden Ansprüche, **dadurch gekennzeichnet, daß** ein Kernelement (112, 412) innerhalb der Kammer angeordnet ist, wobei das Kernelement sich zwischen einer Endwand (117, 417) der Kammer zu einer Stelle in der Nähe der einzelnen Durchlaßbegrenzung (110) erstreckt.

5. Inhaliervorrichtung nach Anspruch 4, **dadurch gekennzeichnet, daß** das Kernelement einen kegelstumpfförmigen Abschnitt (113) hat, dessen großes Ende nahe der Endwand (117, 417) der Kammer liegt und einen zylindrischen Abschnitt (114) konstanten Querschnitts, der sich von dem kleineren Ende des kegelstumpfförmigen Abschnitts hoch zu dem Bereich der Durchlaßbegrenzung (110) erstreckt.

6. Inhaliervorrichtung nach einem der vorstehenden Ansprüche, **dadurch gekennzeichnet, daß** ein Magazin (420, 421) zum Halten mehrerer Pulvergaben, von denen jede vorzugsweise innerhalb einer auftrennbaren Kapsel (422) beinhaltet ist, an der Kammer befestigt ist, und daß das Magazin mit dem Inneren der Kammer mittels einer in der Wand der Kammer (105, 405) vorgesehenen Einlaßöffnung, verbunden ist.

7. Inhaliervorrichtung nach einem der vorstehenden Ansprüche, **dadurch gekennzeichnet, daß** zumindest die Kammer (105, 405) der Inhaliervorrichtung aus transparentem Material hergestellt ist.

8. Inhaliervorrichtung nach einem der vorstehenden Ansprüche, **dadurch gekennzeichnet, daß** ein vorstehender Abschnitt (111, 411) an dem Äußeren der Inhaliervorrichtung, in einem Abstand von zwischen etwa 2 cm und 6 cm von der ersten Öffnung (103, 203) vorgesehen ist.

9. Inhaliervorrichtung nach einem der Ansprüche 1 bis 8, **dadurch gekennzeichnet, daß** das frei bewegliche Element (106, 206, 406) eine Substanz in Pulverform beinhaltet.

## Revendications

1. Dispositif inhalateur pour substances en poudre, comprenant un élément tubulaire (102, 402) connecté à une chambre (105, 405) contenant une poudre médicamenteuse (8), ledit élément tubulaire étant muni d'une première ouverture (103) en une extrémité, et dans lequel ladite chambre (105, 405) contient en outre un élément (106, 206, 406), de préférence une sphère, qui est dimensionné de manière à occuper une partie mineure de ladite chambre (105, 405), ledit élément (106, 206, 406) étant dimensionné de manière à être librement mobile dans ladite chambre, **caractérisé en ce qu'**une pluralité de passages d'entrée (104, 204) est prévue dans ladite chambre (105, 405), ladite pluralité de passages d'entrée (104, 204) étant agencée substantiellement tangentiellement à ladite chambre (105, 405) de façon à faire tourbillonner de l'air, aspiré par cette voie, autour de ladite chambre, et dans lequel une restriction simple (110) est agencée dans la direction d'inhalation entre la première ouverture (103, 203) et ladite pluralité de passages d'entrée (104, 204).

2. Dispositif inhalateur selon la revendication 1, **caractérisé en ce que** ladite chambre (105, 405) comporte une paroi dont la forme est destinée à munir la chambre d'une première partie (a) de section croissante dans une direction orientée vers ladite restriction et d'une deuxième partie (b) de section décroissante dans une direction orientée vers ladite restriction (110).

3. Dispositif inhalateur selon la revendication 1 ou 2, **caractérisé en ce que** ladite chambre a une paroi intérieure (107) qui est de section sensiblement circulaire.

4. Dispositif inhalateur selon l'une quelconque des revendications précédentes, **caractérisé en ce qu'**un élément central (112, 412) est placé à l'intérieur de ladite chambre, ledit élément central s'étendant entre une paroi d'extrémité (117, 417) de ladite chambre jusqu'à une position proche de la restriction simple (110).

5. Dispositif inhalateur selon la revendication 4, **caractérisé en ce que** ledit élément central comporte une partie tronconique (113) avec sa grande extrémité proche de la paroi d'extrémité (117, 417) de ladite chambre et une partie cylindrique de section constante (114) qui s'étend de la petite extrémité de ladite partie tronconique à la zone de la restriction (110).

6. Dispositif inhalateur selon l'une quelconque des revendications précédentes, **caractérisé en ce qu'**un magasin (420, 421) servant à porter une pluralité de doses de poudre, chacune contenue de préférence dans une capsule frangible (422), est fixé à ladite chambre, et **en ce que** ledit magasin est connecté à l'intérieur de ladite chambre au moyen d'une ouverture d'entrée prévue dans la paroi de ladite chambre (105, 405).

7. Dispositif inhalateur selon l'une quelconque des revendications précédentes, **caractérisé en ce qu'**au moins ladite chambre (105, 405) dudit dispositif inhalateur est faite d'un matériau transparent.

8. Dispositif inhalateur selon l'une quelconque des revendications précédentes, **caractérisé en ce qu'**une partie saillante (111, 411) est prévue sur l'extérieur du dispositif inhalateur à une distance allant d'environ 2 cm à 6 cm de ladite première ouverture (103, 203).

9. Dispositif inhalateur selon l'une quelconque des revendications 1 à 8, **caractérisé en ce que** ledit élément librement mobile (106, 206, 406) contient une substance sous forme de poudre.
